**Europäisches Patentamt**

**European Patent Office**

**Office européen des brevets**

(19)

(11) Veröffentlichungsnummer : **0 020 938**
**B1**

(12) **EUROPÄISCHE PATENTSCHRIFT**

(45) Veröffentlichungstag der Patentschrift :
05.10.83

(51) Int. Cl.³ : **G 01 N 27/56**

(21) Anmeldenummer : **80102284.9**

(22) Anmeldetag : **26.04.80**

(54) Polarographischer Messfühler für die Bestimmung des Sauerstoffgehaltes in Gasen, insbesondere in Abgasen von Verbrennungsmotoren.

(30) Priorität : 09.06.79 DE 2923483

(43) Veröffentlichungstag der Anmeldung :
07.01.81 Patentblatt 81/01

(45) Bekanntmachung des Hinweises auf die Patenterteilung : 05.10.83 Patentblatt 83/40

(84) Benannte Vertragsstaaten :
DE FR GB IT SE

(56) Entgegenhaltungen :
DE A 2 300 417
DE A 2 454 339
DE A 2 654 483
DE A 2 654 892

(73) Patentinhaber : ROBERT BOSCH GMBH
Postfach 50
D-7000 Stuttgart 1 (DE)

(72) Erfinder : Müller, Klaus, Dr.
Schillerstrasse 42
D-7144 Tamm (DE)
Erfinder : Maurer, Helmut
Hermann-Essig-Strasse 104
D-7141 Schwieberdingen (DE)
Erfinder : Dietz, Hermann, Dr. Dipl.-Chem.
Steinbeisstrasse 48
D-7016 Gerlingen (DE)
Erfinder : Friese, Karl-Hermann, Dr. Dipl.-Phys.
Strohgäu Strasse 13
D-7250 Leonberg (DE)
Erfinder : Leibfried, Wolfgang, Dr. Dipl.-Phys.
Obere Burghalde 20
D-7250 Leonberg (DE)
Erfinder : Stecher, Günther, Dipl.-Phys.
Seestrasse 64
D-7140 Ludwigsburg (DE)
Erfinder : Linder, Ernst, Dipl.-Ing.
Uhlandstrasse 24
D-7130 Mühlacker (DE)

## Polarographischer Meßfühler für die Bestimmung des Sauerstoffgehaltes in Gasen, insbesondere in Abgasen von Verbrennungsmotoren

### Stand der Technik

Die Erfindung geht aus von einem polarographischen Meßfühler nach der Gattung des Anspruchs 1 oder 2. Aus der DE-A 26 54 483 ist bereits ein derartiger Meßfühler bekannt, der einen vom Meßgas abgetrennten Kathodenraum aufweist, der über eine Öffnung mit dem zu messenden Gas in Berührung steht. Durch Variation der Öffnungsgröße läßt sich die Diffusion an die Elektrodengröße anpassen. Bei zu großer Öffnung ändert sich jedoch der gemessene Strom praktisch nicht mehr in Abhängigkeit von der Sauerstoffkonzentration, da der Meßfühler dann nicht mehr im Diffusionsgrenzstrom-Bereich arbeitet. Der Nachteil der bekannten Vorrichtung ist jedoch vor allem darin zu sehen, daß aufgrund des verhältnismäßig großen Kathodenraumes sich dieser bei Konzentrationswechseln wie eine träge Mischkammer verhält und die Ansprechgeschwindigkeit eines solchen Meßfühlers daher sehr klein ist. Außerdem sind derartige Meßfühler verhältnismäßig groß und kompliziert herzustellen.

### Vorteile der Erfindung

Der erfindungsgemäße Meßfühler mit den Merkmalen des Anspruchs 1 bzw. des Anspruchs 2 hat demgegenüber den Vorteil, daß er nur sehr kleine Zwischenräume im Bereich der Kathode aufweist und daher im Zusammenwirken mit den im Vergleich zu ihrem Durchmesser langen Löchern auf Konzentrationswechsel des Sauerstoffgehaltes sehr schnell anspricht. Außerdem läßt er sich in einer sehr kompakten Form einfach herstellen.

Durch die in den abhängigen Ansprüchen aufgeführten Maßnahmen sind vorteilhafte Weiterbildungen und Verbesserungen des im Hauptanspruch angegebenen Meßfühlers möglich, wobei es besonders vorteilhaft ist, sich für die Herstellung des Meßfühlers der Siebdrucktechnik zu bedienen.

### Zeichnung

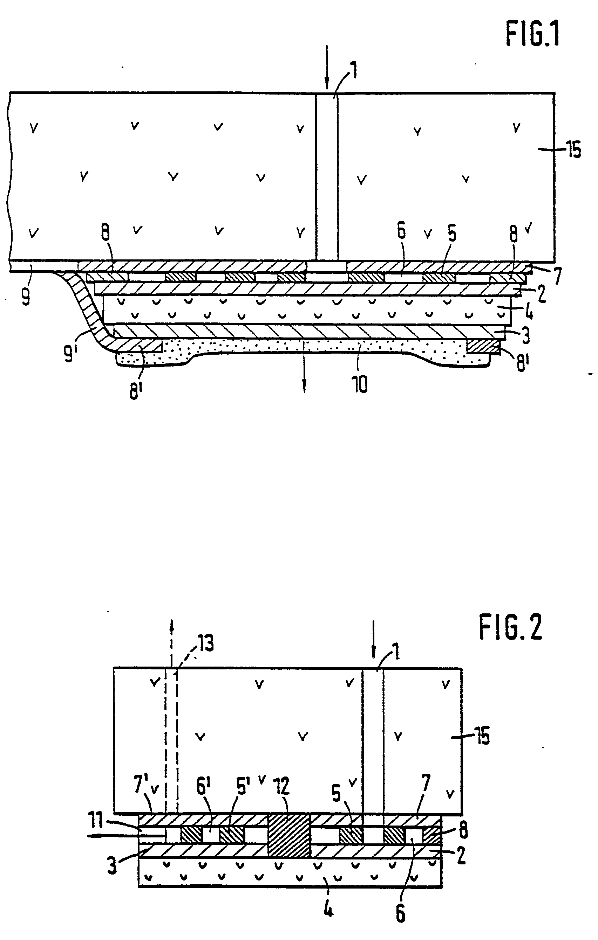

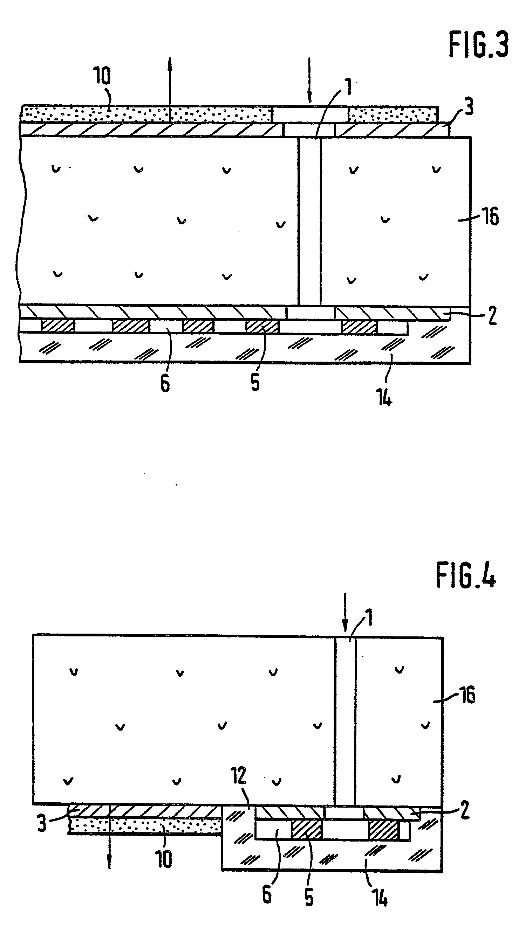

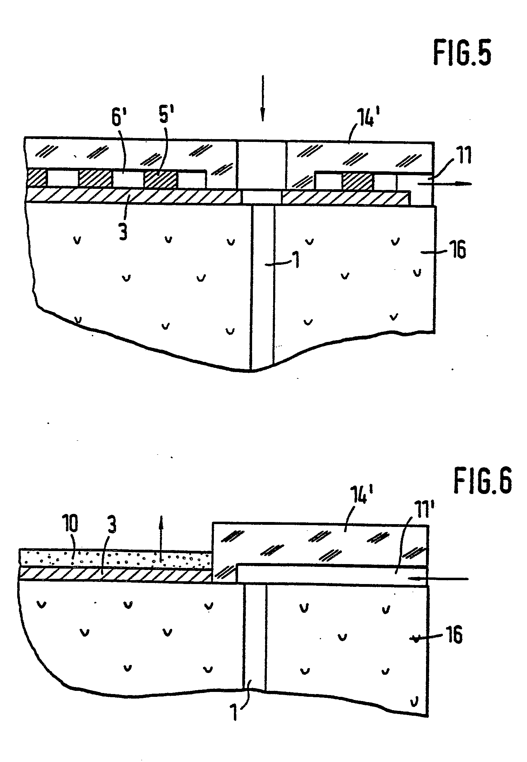

Ausführungbeispiele der Erfindung sind in der Zeichnung dargestellt und in der nachfolgenden Beschreibung näher erläutert. Es zeigen die Figuren 1 und 2 Schnitte durch Ausführungsbeispiele, bei denen der Meßfühler auf einem elektrisch neutralen keramischen Plättchen aufgebaut wird, während die Figuren 3 bis 6 Ausführungsbeispiele zeigen, bei denen das Trägerplättchen gleichzeitig den Elektrolyten bildet, wobei die Figuren 5 und 6 Verbesserungen des Ausführungsbeispiels nach Fig. 3 zeigen.

### Beschreibung der Ausführungsbeispiele

Der in Fig. 1 dargestellte Meßfühler weist ein Trägerplättchen 15 auf, das ca. 4 × 5 mm groß ist und eine Dicke von 1,2 mm hat. Dieses Trägerplättchen 15 besteht aus einem dichten Keramikmaterial, z. B. aus Aluminiumoxid, und weist ein Loch 1 von ca. 50 $\mu$m Durchmesser auf. Statt eines einzigen Loches auf der angegebenen Fläche können auch mehrere Löcher von einem Durchmesser von 10 bis 20 $\mu$m vorgesehen werden, wobei etwa ein Loch/mm$^2$ vorgesehen werden muß. Das Trägerplättchen 15 kann alternativ auch aus einer grobporigen Keramik bestehen, die dann mit einer entsprechende Löcher aufweisenden dichten Glasschicht abgedeckt sein muß. Der Meßfühler weist weiter eine Kathode 2 und eine Anode 3 mit einem dazwischenliegenden Elektrolyten 4 auf. Die Elektroden 2 und 3 bestehen entweder aus Platin oder aus einem Gemisch aus Platin und stabilisiertem Zirkondioxid, wobei dieses Zirkondioxid etwa 40 Vol.-% ausmacht. Der Elektrolyt 4 besteht aus in bekannter Weise stabilisiertem Zirkondioxid. Zwischen der Kathode 2 und dem Trägerplättchen 15 befinden sich vorzugsweise quadratische Stützen 5 in Form eines Rasters, so daß zwischen diesen Stützen 5 Zwischenräume 6 gebildet werden. Die Stützen 5 bestehen vorzugsweise aus dem gleichen Material wie die Kathode 2, so daß die Mantelflächen dieser Stützen 5 eine zusätzliche Elektrodenfläche schaffen. Die Zwischenräume 6 stehen mit dem Loch 1 in Verbindung, so daß über das Loch 1 und die Zwischenräume 6 Sauerstoff an die Kathode 2 gelangen kann. Zur gleichmäßigen Versorgung der Kathode 2 mit Elektronen ist es vorteilhaft, eine Platinschicht 7 zwischen den Stützen 5 und dem Trägerplättchen 15 anzuordnen. Eine weitere Verbesserung läßt sich durch die Anordnung eines Platinrahmens 8 am Umfang der Kathode 2 erzielen. Ein ebensolcher Rahmen 8' kann auch auf der Anode 3 angebracht werden. Beide Rahmen 8 beziehungsweise 8' münden in Leiterbahnen 9 bzw. 9', an welche die für den Betrieb des Meßfühlers notwendige Spannung angelegt wird. Auf der dem Elektrolyten entgegengesetzten Seite der Anode 3 wird eine poröse, keramische Schutzschicht 10, z. B. aus porösem Aluminiumoxid, aufgebracht, durch die der an der Anode gebildete Sauerstoff entweichen kann.

Das zu messende Gas steht mit der Oberseite des Trägerplättchens 15 in Verbindung. Das Gas gelangt durch das Loch 1 in die Zwischenräume 6 und von dort an die Kathode, wo durch die angelegte Spannung an der Dreiphasengrenze Kathode/Elektrolyt/Gas der Sauerstoff reduziert wird und in Form von Sauerstoffionen durch den Elektrolyten 4 hindurch an die Anode 3 gelangt, wo die Sauerstoffionen wieder zu Sauerstoffmolekülen oxidiert werden, die durch die poröse Schutzschicht 10 hindurch entweichen. Die zwischen Kathode und Anode durch den Elektrolyten hindurchwandernden Sauerstoffionen verursachen einen Stromfluß, dessen Größe von der Menge der an der Kathode ankommenden Sauerstoffmoleküle abhängig ist, solange der Meßfühler im Bereich des Diffusionsgrenzstromes arbeitet.

Während das Ausführungsbeispiel nach Fig. 1 eine sogenannte bilaterale Lösung zeigt, bei der Kathode 2 und Anode 3 auf je einer Seite des Elektrolyten 4 angeordnet sind, zeigt die Fig. 2 eine sogenannte unilaterale Anordnung, bei der Kathode 2 und Anode 3 auf der gleichen Seite des Elektrolyten 4 angeordnet sind. Eine Zwischenwand 12 aus einer dichten, nicht leitenden Keramik, z. B. Aluminiumoxid, trennt den Gasraum der Kathode 2 (rechte Hälfte in Fig. 2) von Gasraum der Anode 3 (linke Hälfte der Fig. 2). Wie im Ausführungsbeispiel nach Fig. 1 gelangt der Sauerstoff durch das Loch 1 in dem Trägerplättchen 15 in die durch die Stützen 5 gebildeten Zwischenräume 6 und erhält so Kontakt mit der Kathode 2. Die an der Kathode 2 gebildeten Sauerstoffionen wandern über den Elektrolyten 4 an die Anode 3, wo sie wiederum zu molekularem Sauerstoff oxidiert werden, der über die durch die Stützen 5' gebildeten Zwischenräume 6' des Anodenraumes entweder über einen Kanal 11 oder über ein Loch 13 entweichen kann. Der Rahmen 8 dichtet den Gasraum der Kathodenseite zum Rand hin ab, so daß das Gas nur über die Bohrung 1 eindiffundieren kann. Es ist, wie bei dem Ausführungsbeispiel nach Fig. 1, auch hier vorteilhaft, zwischen dem Trägerplättchen 15 und den Stützen 5 bzw. 5' eine Platinschicht 7 bzw. 7' anzuordnen.

Bei den Ausführungsbeispielen nach den Figuren 3 bis 6 übernimmt ein Trägerplättchen 16 gleichzeitig die Funktion des Elektrolyten, so daß kein extra Elektrolyt zwischen Kathode und Anode vorgesehen werden muß. Die Elektrodenanordnung nach Fig. 3 ist wiederum bilateral. Der Sauerstoff gelangt jedoch nicht von der Kathodenseite (Unterseite in Fig. 3) an die Kathode 2, sondern von der Anodenseite (Oberseite in Fig. 3) durch das Loch 1, mit welchem auch die Anode 3 und die Kathode 2 versehen sind. Der Sauerstoff verteilt sich über die Zwischenräume 6 auf die Kathode 2. Die Zwischenräume 6 werden gebildet aus den Stützen 5, die wiederum aus dem Elektrodenmaterial bestehen, und einer gasdichten Abdeckung 14, die z. B. aus einem Glas besteht. Zwischen den Stützen 5 und der Abdeckung 14 kann wiederum eine in der Figur nicht dargestellte Platinzwischenschicht vorgesehen werden, die einer guten Elektronenversorgung der Kathode dient. Ebenso können für die Kathode 2 und die Anode 3 Platinrahmen vorgesehen werden, wie sie in Fig. 1 dargestellt sind. Es ist vorteilhaft, wenn in jedem Falle die Anode 3 durch eine poröse keramische Schutzschicht 10 abgedeckt ist. Durch diese Schutzschicht 10 kann dann der an der Anode entstehende Sauerstoff entweichen. — Fig. 4 stellt die entsprechende unilaterale Ausführungsform dar. Auch hier dient das Trägerplättchen 16 als Elektrolyt. Kathode 2 und Anode 3 befinden sich auf einer Seite des Elektrolyten 16 und sind durch eine gasdichte Zwischenwand 12 voneinander getrennt, wobei die Zwischenwand 12 einen Teil der gasdichten Abdeckung 14 darstellt, die zusammen mit den Stützen 5 die Zwischenräume 6 bildet. Der Sauerstoff diffundiert durch das Loch 1 im Elektrolyten 16 und in der Kathode 2 in die Zwischenräume 6, wird an der Dreiphasengrenze Gas/Kathode 2/Elektrolyt 16 reduziert und gelangt in Form von Sauerstoffionen über den Elektrolyten 16 zur Anode 3, wird dort wieder zu molekularem Sauerstoff oxidiert und diffundiert durch die poröse Schutzschicht 10. Vorteil dieser Ausführungsform nach Fig. 4 ist der, daß eine Vermischung von eindiffundierendem und austretendem Sauerstoff vermieden wird, da der Sauerstoff von der einen Seite des Elektrolyten 16 eindiffundiert und nach der anderen Seite des Elektrolyten hin abgegeben wird. Dies kann auch bei der bilateralen Ausführungsform nach Fig. 3 erreicht werden, wie dies in den Figuren 5 und 6 dargestellt ist. Gemäß Fig. 5 werden auf die Anode der Fig. 3 Stützen 5' sowie — unter Aussparung einer Öffnung für das Loch 1 — eine gasdichte Abdeckung 14' unter Bildung von Zwischenräumen 6' aufgebracht, derart, daß der an der Anode 3 gebildete Sauerstoff durch einen Kanal 11 entweichen kann. Gemäß Fig. 6 kann eine Trennung von eindiffundierendem und abgegebenem Sauerstoff bei der bilateralen Ausführungsform nach Fig. 3 auch dadurch erreicht werden, daß man durch eine gasdichte Abdeckung 14' einen Tunnel 11', der von der Vorderkante des Elektrolyten 16 zu dem Loch 1 führt, vorsieht. In diesem Bereich ist die Anode 3 mit ihrer Schutzschicht 10 ausgespart.

**Ansprüche**

1. Polarographischer Meßfühler für die Bestimmung des Sauerstoffgehaltes in Gasen, insbesondere in Abgasen von Verbrennungsmotoren, der nach dem Diffusionsgrenzstrom-Prinzip arbeitet, mit einem Festelektrolytkörper (4), der eine schichtförmige Anode (3) und eine schichtförmige Kathode (2) trägt, an die eine konstante Spannung anlegbar ist, wobei der Kathode eine Diffusionsbarriere in Form mindestens eines Loches (1) in einer die Kathode vom zumessenden Gas trennenden Wand (15) vorgeordnet ist, dadurch gekennzeichnet, daß auf der Kathode (2) Stützen (5) aufliegen, die zwischen der Kathode (2) und der Wand (15) ein System von Zwischenräumen (6) bilden, daß diese Zwischenräume (6) über das Loch (1) mit dem sauerstoffhaltigen Gas in Verbindung stehen, und daß das Loch eine im Vergleich zu seinem Durchmesser große Länge hat.

2. Polarographischer Meßfühler für die Bestimmung des Sauerstoffgehaltes in Gasen, insbesondere in Abgasen von Verbrennungsmotoren, der nach dem Diffusionsgrenzstrom-Prinzip arbeitet, mit einem Festelektrolytkörper (16), der eine schichtförmige Anode (3) und eine schichtförmige Kathode (2) trägt, an die eine konstante Spannung anlegbar ist, wobei der Kathode eine Diffusionsbarriere in Form mindestens eines Loches (1) in einer die Kathode vom zumessenden Gas trennenden Wand vorgeordnet ist, dadurch gekennzeichnet, daß die Wand von dem Festelektrolytkörper (16) gebildet ist, daß auf der dem Festelektrolytkörper abgewandten Seite der Kathode (2) Stützen (5) aufliegen, die zwischen der Kathode (2) und einer weiteren, die Kathode gasdicht umgebenden Wand (14) ein System von Zwischenräumen (6) bilden, daß diese Zwischenräume (6) über das Loch (1) mit dem sauerstoffhaltigen Gas in Verbindung stehen, und daß das Loch (1) eine im Vergleich zu seinem Durchmesser große Länge hat.

3. Meßfühler nach Anspruch 1 oder 2, dadurch gekennzeichnet, daß die Stützen (5) einen quadratischen Stützquerschnitt aufweisen.

4. Meßfühler nach Anspruch 1 oder 2, dadurch gekennzeichnet, die Diffusionsbarriere mehrere Löcher umlaßt und daß die Löcher (1) einen Durchmesser von 10 bis 60 µm und eine Länge von mehr als 1 mm und die Zwischenräume (6) eine Höhe von 10 bis 50 µm und eine Erstreckungzwischen den Stützen von 0,2 bis 0,5 mm aufweisen.

5. Meßfühler nach einem der Ansprüche 1 bis 3, dadurch gekennzeichnet, daß die Stützen (5) aus elektronenleitendem Material, vorzugsweise aus dem gleichen Material wie die Kathode (2) und die Anode (3) bestehen.

6. Meßfühler nach Anspruch 4, dadurch gekennzeichnet, daß die Kathode (2) und die Anode (3) und die Stützen (5) aus Platin oder einem Platin-Zirkondioxid-Gemisch bestehen.

7. Meßfühler nach Anspruch 1, dadurch gekennzeichnet, daß die Stützen (5) auf der der Kathode (2) abgewandten Seite eine alle Stützen überdeckende Platinschicht (7) aufweisen, die eine mit dem Loch (1) kommunizierende Öffnung aufweist.

8. Meßfühler nach einem der vorhergehenden Ansprüche, dadurch gekennzeichnet, daß die Kathode (2) an ihrem äußeren Umfang einen Rahmen (8) aus Platin aufweist.

9. Meßfühler nach Anspruch 8, dadurch gekennzeichnet, daß auch über der Anode (3) mit Hilfe von weiteren Stützen (5') und einer über diesen angeordneten Abdeckwand (15 ; 14') Zwischenräume (6') und ein Gas-Austrittskanäl (11) gebildet sind.

10. Meßfühler nach Anspruch 8, dadurch gekennzeichnet, daß die Gaseintrittsseite des Lochs (1) mit einem zum Rande des Elektrolytkörpers (16) führenden Zuleitungskanal (11') kommuniziert, der innerhalb einer auf der Außenseite des Elektrolytkörpers angeordneten Deckschicht (14') vorgesehen ist.

## Claims

1. A polarographic sensor for determining the oxygen content in gases, especially in exhaust gases of internal combustion engines, which operates in accordance with the diffusion limit current principle, comprising a body of solid electrolyte (4) which supports an anode (3) in the form of a layer and a cathode (2) in the form of a layer to which a constant voltage can be applied, wherein the cathode is arranged in front of a diffusion barrier in the form of at least one hole (1) in a wall (15) separating the cathode from the gas to be monitored, characterised in that, supports (5) rest upon the cathode (2) and form a system of intermediate spaces (6) between the cathode (2) and the wall (15), that these intermediate spaces (6) are in communication with the oxygen containing gas through the hole (1) and that the hole is of great length in comparison to its diameter.

2. A polarographic sensor for determining the oxygen content in gases, especially in exhaust gases of combustion engines, which operates in accordance with the diffusion limit current principle, comprising a body (16) of solid electrolyte which supports an anode (3) in the form of a layer and a cathode (2) in the form of a layer to which a constant voltage can be applied, wherein the cathode is arranged in front of a diffusion barrier in the form of at least one hole (1) in a wall separating the cathode from the gas to be monitored, characterised in that, the wall is formed by the body (16) of solid electrolyte, that supports (5) rest on the side of the cathode (2) remote from the body of solid electrolyte, and form a system of intermediate spaces (6) between the cathode (2) and a further wall (4) surrounding the cathode gas-tight, that these intermediate spaces (6) are in communication with the oxygen containing gas through the hole (1) and that the hole (1) is of great length in comparison to its diameter.

3. A sensor according to claim 1 or 2, characterised in that, the supports (5) have a square supporting crosssection.

4. A sensor according to claim 1 or 2, characterised in that, the diffusion barrier comprises a plurality of holes and that the holes (1) have a diameter of 10 to 60 µm and a length of more than 1 mm and the intermediate spaces (6) have a height of 10 to 50 µm and a length between the supports of 0.2 to 0.5 mm.

5. A sensor according to one of claims 1 to 3, characterised in that, the supports (5) consist of electron conducting material, preferably of the same material as the cathode (2) and the anode (3).

6. A sensor according to claim 4, characterised in that, the cathode (2) and the anode (3) and the supports (5) consist of platinum or a platinum-zirconium-dioxide mixture.

7. A sensor according to claim 1, characterised in that, on the side remote from the cathode (2), the supports (5) have a platinum layer (7) covering all the supports and having an opening communicating with the hole (1).

8. A sensor according to one of the preceding claims, characterised in that, at its outer periphery, the cathode (2) has a frame (8) of platinum.

9. A sensor according to claim 8, characterised in that, intermediate spaces (6') and a gas outlet duct (11) are also formed over the anode (3) with the aid of further supports (5') and a cover wall (15 ; 14') arranged above the supports.

10. A sensor according to claim 8, characterised in that, the gas inlet side of the hole (1) communicates with a supply duct (11'), leading to the edge of the body (16) of electrolyte, and which is provided within a cover layer (14') arranged on the outside of the body of electrolyte.

## Revendications

1. Détecteur polarographique pour déterminer la teneur en oxygène dans des gaz, notamment dans des gaz d'échappement de moteurs à combustion, détecteur fonctionnant selon le principe du courant limite de diffusion, avec un corps d'électrolyte solide (4), portant une anode (3) en forme de couche et une cathode (2) en forme de couche, auxquelles est susceptible d'être appliquée une tension constante, une barrière de diffusion, revêtant la forme d'au moins un trou (1) dans une paroi (15) séparant la cathode du gaz à mesurer, étant disposée en avant de la cathode, détecteur caractérisé en ce que, sur la cathode (2) sont posés des supports (5) qui forment entre la cathode (2) et la paroi (15) un système d'espaces intermédiaires (6), ces espaces intermédiaires (6) étant en communication avec le gaz contenant de l'oxygène par l'intermédiaire du trou (1), ce trou ayant une grande longueur par comparaison avec son diamètre.  •

2. Détecteur polarographique pour déterminer la teneur en oxygène dans des gaz, notamment dans des gaz d'échappement de moteurs à combustion, détecteur fonctionnant selon le principe du courant limite de diffusion, avec un corps d'électrolyte solide (4), portant une anode (3) en forme de couche et une cathode (2) en forme de couche, auxquelles est susceptible d'être appliquée une tension constante, une barrière de diffusion, revêtant la forme d'au moins un trou (1) dans une paroi (15) séparant la cathode du gaz à mesurer, étant disposée en avant de la cathode, détecteur caractérisé en ce que la paroi est formée par le corps d'électrolyte solide (16), en ce que des supports (5) reposent sur la face, opposée au corps d'électrolyte solide de la cathode (2), ces supports formant entre la cathode (2) et une autre paroi (14) entourant la cathode, un système d'espaces intermédiaires (6), ces espaces intermédiaires (6) étant en communication avec le gaz contenant de l'oxygène par l'intermédiaire du trou (1), et ce trou (1) ayant une grande longueur en comparaison de son diamètre.

3. Détecteur selon la revendication 1 ou 2, caractérisé en ce que les supports (5) présentent une coupe transversale d'appui carrée.

4. Détecteur selon la revendication 1 ou 2, caractérisé en ce que la barrière de diffusion comprend plusieurs trous et que ces trous (1) ont un diamètre allant de 10 à 60 $\mu$m et une longueur de plus d'un millimètre, tandis que les espaces intermédiaires (6) ont une hauteur de 10 à 50 $\mu$m et une étendue entre les supports de 0,2 à 0,5 mm.

5. Détecteur selon l'une des revendications 1 à 3, caractérisé en ce que les supports (5) sont constitués d'un matériau conducteur d'électrons, de préférence de même matériau que la cathode (2) et que l'anode (3).

6. Détecteur selon la revendication 4, caractérisé en ce que la cathode (2), l'anode (3), et les supports (5), sont constitués de platine ou d'un mélange de platine et de dioxyde de zirconium.

7. Détecteur selon la revendication 1, caractérisé en ce que les supports (5) comportent sur leurs côtés opposés à la cathode (2) une couche de platine (7) recouvrant tous les supports, et qui présente un orifice communiquant avec le trou (1).

8. Détecteur selon l'une des revendications précédentes, caractérisé en ce que la cathode (2) comporte, à sa périphérie externe, un cadre (8) en platine.

9. Détecteur selon la revendication 8, caractérisé en ce que, également au-dessus de l'anode (3) des espaces intermédiaires (6') et un canal de sortie de gaz (11) sont formés à l'aide d'autres supports (5') et d'une paroi de recouvrement (15, 14') disposée au-dessus de ces supports.

10. Détecteur selon la revendication 8, caractérisé en ce que le côté du trou (1) où entre le gaz, communique avec un canal d'amenée (11') aboutissant au bord du corps d'électrolyte solide (16), ce canal étant prévu à l'intérieur d'une couche de recouvrement (14') disposée sur le côté externe du corps d'électrolyte solide.

FIG.1

FIG.2

FIG.3

FIG.4

FIG.5

FIG.6